# EUROPEAN PATENT APPLICATION

(11) **EP 0 827 747 A1**
(43) Date of publication of application: **11.03.1998**
(21) Application number: 97115423.2
(22) Date of filing: 05.09.1997
(51) Int. Cl.: A61K 35/78

(54) **Analgetic agent**

(30) Priority: 05.09.1996 JP 255584/96; 17.10.1996 JP 295649/96
(71) Applicant: Seiwa Pharmaceutical, Limited, Minato-ku, Tokyo (JP)
(72) Inventor: Nakamura, Motoyuki, Kitaibaraki city, Ibaraki (JP); Minagawa, Yumiko, Kitaibaraki city, Ibaraki (JP); Nohara, Toshihiro, Kumamoto city, Kumamoto (JP); Chi, Yu-Ming, Kumamoto city, Kumamoto (JP); Hashimoto, Fumio, Kumamoto city, Kumamoto (JP); Yoshizawa, Toyokichi, Kitaibaraki city, Ibaraki (JP)
(74) Representative: Hansen, Bernd, Dr. Dipl.-Chem.

(57) **Abstract**

An analgetic agent comprising incarvillateine or other monoterpene alkaloid extracted from Incarvillea sinensis Lam. as an effective component shows an analgetic and anti-inflammatory action (first phase) similar to those give by the conventional narcotic analgestics such as morphine, etc. and consequently also shows an analgetic and anti-inflammatory action (second phase) as given by the ordinary analgetics such as aspirin, etc. and also shows a distinguished analgetic and anti-inflammatory action not only on headache, stomachache, neuralgia, etc., but also on cancer pain.

## Description

### BACKGROUND OF THE INVENTION

### 1. FIELD OF THE INVENTION

The present invention relates to an analgetic agent and more particularly an analgetic agent comprising a plant extract as an effective component.

### 2. DESCRIPTION OF RELATED ART

It is an important nursing task to cure various diseases accompanying intolerable pains, particularly cancer pain accompanied by the endostage cancer. Causes for the cancer pain include tumor proper, side effects of cancer-curing agents, psychologic pain, etc., to which a Brompton cocktail and WHO prescription are offen applied.

According to these prescriptions, administration starts from a small dose, which is gradually increased until pain can be substantially completely released, where morphine is used as a basic drug. Continuous administration of such a drug leads to formation of a drug resistance, resulting in formation of considerable mental and physical dependency thereon. Once such a chronic intoxication is formed, abrupt abstinence symptoms appear upon interruption of the administration, resulting in recurrence of pain and deterioration of mental and physical functions such as anxiety, phobias, insomnia, etc. Even if the drug is administered again in the same dose as the last one, no satisfactory effect can be obtained.

Many compounds showing an analgetic and anti-inflammatory action on cancer pain have been so for reported. Particularly popular crude drug showing an analgetic and anti-inflammatory action is opium poppy (Papaver somuniferum). Though the opium poppy has good analgetic, antispastic, antitussive and constipating actions, it is designated as narcotic due to its strong dependency. Main components of opium extracted from the opium poppy are morphine-based opium alkaloids and morphine derivatives, and various side effects inevitably appear due to their nature in case of continuous administration thereof, as mentioned above.

### SUMMARY OF THE INVENTION

An object of the present invention is to provide an analgetic agent without any substantial side effects, which comprises a plant extract as an effective component.

The object of the present invention can be attained by an analgetic agent, which comprises an organic solvent or water extract of Incarvillea sinensis Lam. as an effective component.

### DETAILED DESCRIPTION OF THE INVENTION

Incarvillea sinensis Lam. is a plant of trumpet creeper family (Bignoniaceae), which is considered to have a cold or rheumatism-curing action and also have an antidotal action, and is used as a folk medicine in China. In the present invention, extraction is made of its whole plant, but it has not been so far known at all that its extract has an analgetic and anti-inflammary action.

Extraction of the whole plant of Incarvillea sinensis Lam. can be made from crude plants or dried and pulverized plants, using an organic solvent or water including hot water. A combination of an organic solvent and water may be used, when required. Organic solvents for extraction includes, for example, methanol, ethanol, n-butanol, ethyl acetate, chloroform, etc., among which methanol and ethanol are preferable. Extraction with these extracting solvents can be carried out at a temperature ranging from room temperature to reflux temperature.

Among these extracts, a component having a particularly remarkable analgetic and anti-infammatory action is a 60% methanol eluate fraction (Sample 6 as will be described later) of methanol extracts by polystyrene-based column chromatography, and incarvillateine is isolated from the eluate fraction as a compound having a good analgtetic and anti-inflammatory action. It is well known that incarvillateine itself is a compound isolated from 95% ethanol extract of Incarvillea sinensis Lam., but it is the present inventors that have found for the first time that the compound has a distinguished analgetic and anti-inflammatory action.

The analgetic and anti-inflammatory action can be observed among other monoterpene alkaloids extracted from Incarvillea sinensis Lam., together with incarvillateine. Such monoterpene alkaloids include the following compounds:
Incarvilline
Oxyincarvilline
Incarvine A or its N-oxide
Incarvine B
Incarvine C
Incarvine D
Incarvillateine N-oxide
3''-methoxyincarvillateine

Incarvillateine and its derivatives are represented by the following chemical formula: 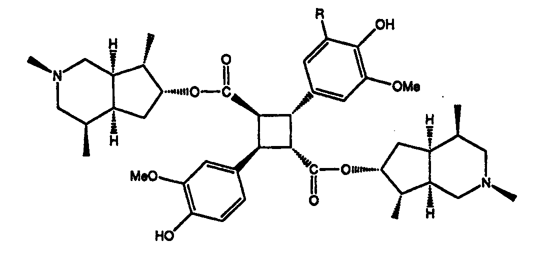
- R=H: Incarvillateine
- R=OMe: 3''-methoxyincarvillateine
where R=H provides incarvillateine and R=OMe provides 3''-methoxyincarvillateine.

Organic solvent extracts or water extracts of Incarvillea sinensis Lam., particularly organic solvent extracts of Incarvillea sinensis Lam. containing incarvillateine, other monoterpene alkaloids or these compounds together can be formulated in the form of powder, granules, tablets, sugar-coated tablets, capsules, liquid preparation, etc. and can be administered orally, parenterally, by inhalation, transrectally, locally, etc. Parenteral administration includes, for example, subcutaneous injection, intravenous injection, intramuscular administration, nasal administration or infusion, etc. Dose is usually in a range of about 0.1 to about 200 mg/kg of body weight per administration. Exact dose is selected from the above-mentioned range usually in view of age, body weight, symtom of patients, administration route, etc., and administration is carried out 1 to 5 times in a day. Their toxicity is low, and investigation on wistar male rats for acute toxicity by oral admininistration showed no case of death even at a dose of 3,000 mg/kg(p.o.).

The present invention provides an analgetic agent comprising incarvillateine or other monoterpene alkaloids extracted from Incarvillea sinensis Lam. as an effective component. The above-mentioned plants are used originally as a folk medicine and thus has no fear of chronic intoxication. Furthermore, the analgetic and anti-inflammatory action given by the present analgetic agent is a similar action (first phase) to the analgetic and anti-inflammatory action given by the conventional narcotic analgetic such as morphine, etc. and consequently is also an analgetic and anti-inflammatory action (secound phase) given by the ordinary analgetic such as aspirin, etc. Thus, the present analgetic agent has a distinguished analgetic and anti-inflammatory action not only on headache, stomachache, neuralgia, etc., but also on cancer pain.

### BRIEF DESCRIPTION OF DRAWINGS

Fig. 1 is a diagram showing results of analgetic and anti-inflammatory action of Samples Nos. 2 to 8, when intraperitoneally administered (formalin test).

Fig. 2 is a diagram showing results of analgetic and anti-inflammatory action of Sample No.9, when intraperitoneally administered (formalin test).

Fig. 3 is a diagram showing results of motor activity by Sample No. 9, when intraperitoneally administered.

### PREFERRED EMBODIMENTS OF THE INVENTION

The present invention will be described in detail below, referring to Examples.

### EXAMPLES 1

1.25kg of dried Incarvillea sinensis Lam. was extracted with 6.5 liters of methanol at room temperature, and its residues were also extracted with 5.5 liters of methanol at room temperature. The extract methanol solutions were joined together and concentrated under reduced pressure, whereby 42.7g of dark green powdery extract was obtained (Sample No. 1).

Then, Sample No. 1 was subjected to column chromatography using 230 ml of polystyrene-based resin (Diaion HP-20, styrene/divinylbenzene-based copolymer resin, made by Mitubishi Kagaku K.K., Japan), whereby 2 liters each of 10% and 30% methanol eluate fractions, and 3 liters each of 40%, 50%, 60%, 70% and 80% methanol eluate fractions were obtained. The eluates each were concentrated under reduced pressure, whereby the following Samples Nos. 2 to 8 were obtained correspondingly.
- Sample No. 2:: 10% methanol eluate fraction 1.7g
- Sample No. 3:: 30% methanol eluate fraction 0.6g
- Sample No. 4:: 40% methanol eluate fraction 1.0g
- Sample No. 5:: 50% methanol eluate fraction 1.4g
- Sample No. 6:: 60% methanol eluate fraction 1.5g
- Sample No. 7:: 70% methanol eluate fraction 1.9g
- Sample No. 8:: 80% methanol eluate fraction 0.7g

### Formalin Test (Test for determining an analgetic and anti-inflammatory action)

(1) 20 µl of 2% formalin solution was injected as a stimulant to the dorsal surface of a right hind paw (foot sole) of 3.5 week-old male ICR mice adapted to the quarantine for one week after purchase, and motions of mouse to lick the right hind paw back leg to release the pain were measured with a stopwatch continuously for 30 minutes with time segments each of 5-minutes. In that case, 100 µl each of suspensions in aqueous 0.5% solutions of polyoxyethylenesorbitan monooleate (Tween 80, made by Atlas Co.)containing test drugs (Samples Nos. 2 to 8), respectively, prepared to correspond to a dose of 120 mg/kg of test drugs were intraperitoneally administered to mice at the time of 15 minutes before the stimulation. On the other hands, the same volume of an aqueous 0.5% Tween 80 solution was intraperitoneally administered to the control group.

In the formalin test, two phases, i.e., first phase and second phase appear.

The first phase refers to pains appearing for the period of 0 to 10 minutes after the formalin stimulation, that is, pains appearing by direct stimulation of terminals of sensory nerves by formal in injection. Those having an action to suppress the pains are up to now only narcotic analgetics such as morphine, etc., and it seems that their function and mechanism are an action on the central nerve.

Second phase refers to pains appearing for the period of 10 to 30 minutes after the formal in stimulation, that is, pains due to infammations caused by formalin injection. The second phase can be suppressed by ordinary analgetics such as aspirin, etc.

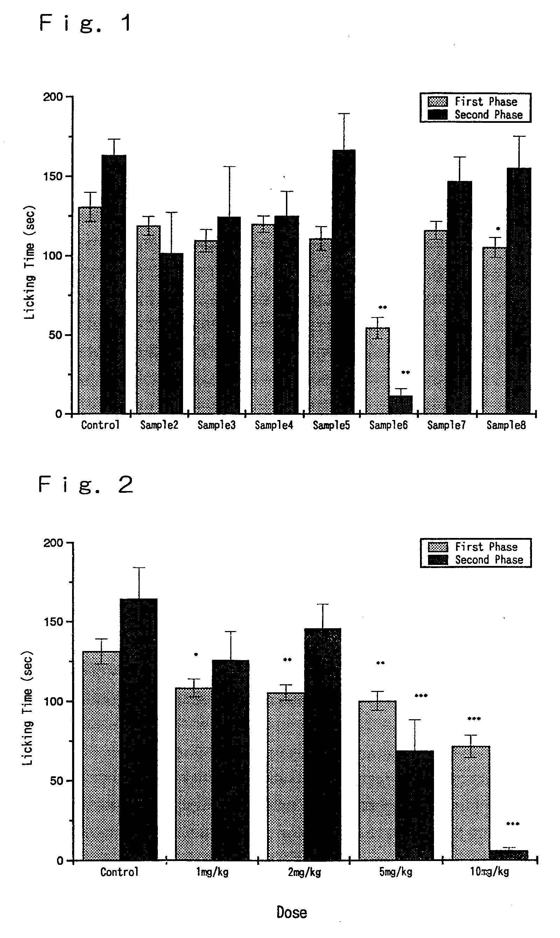

Thus, evaluation was made in these two phases, i.e., first phase for the period of 0 to 10 minutes and secound phase for the period of 10 to 30 minutes by measuring a licking time, i.e. the time during which mice licked the right hind paw, in total second and by comparing the licking time with that of the control group. Results (n=10, average ± standard deviation) are shown in the following Table 1 and Fig. 1.

**Table 1**

| Sample | First phase of 0∼10 min. (sec.) | Second phase of 10∼30 min. (sec.) |
|---|---|---|
| Control | 130.69±9.26 | 163.22±10.28 |
| Sample No. 2 | 118.70±5.98 | 101.47±25.70 |
| Sample NO. 3 | 109.52±7.10 | 124.49±31.74 |
| Sample No. 4 | 119.95±5.04 | 125.01±15.53 |
| Sample No. 5 | 110.91±7.68 | 166.75±22.68 |
| Sample No. 6 | 54.38±6.63 ** | 11.52±4.42 ** |
| Sample No. 7 | 116.16±5.61 | 146.90±15.40 |
| Sample No. 8 | 105.27±6.37 * | 155.49±19.97 |

| | | |
|---|---|---|
| * P<0.05 | | |
| ** P<0.01 | | |

### EXAMPLE 2

280.3mg of Sample No. 6 having a high activity in Example 1 was subjected to column chromatography, using 500 ml of polysaccharide dextran-based beads (Sephadex LH-20, made by Pharmacia LKB Biotechnology Co.), and then to elution with 30% methanol, whereby 5 ml each of fractions Nos. 1 to 20 was obtained.

71.7mg of colorless powder (Sample No. 9) was obtained from fractions Nos. 8 to 10, and subjected to repeated recrystallization from methanol, whereby colorless plate crystals having the following physico-chemical properties were obtained:
m.p. : 217.2∼217.7°C
[α]_{D}:-10.8° (c=0.79, CHCl₃)
EI-MS(m/z):718[M]⁺
¹³C-NMR(CDCl₃-d)δ:171.9(s), 171.7(s), 146.9(s), 146.8(s), 145.5(s), 145.3(s), 130.4(s), 130.2(s), 120.3(d), 119.8(d), 114.7(d), 114.7(d), 110.9(d), 110.8(d), 76.5(d), 76.2(d), 57.4(t), 57.3(t), 57.1(t), 57.1(t), 55.7(q), 55.6(q), 47.8(d), 47.2(d), 46.0(q), 45.9(q), 45.7(s), 45.6(s), 41.7(d), 40.3(d), 40.1(d), 40.1(d), 37.2(s), 37.2(s), 30.1(d), 30.1(d), 29.6(t), 29.1(t), 17.0(q), 16.8(q) 14.8(q), 14.4(q)

These physico-chemical properties were found to be identical with those of incarvillaleine, a compound previously isolated from 95% ethanol extract of the same plant, determined for the structure and disclosed in Phytochemistry, Vol. 29, No. 7, pp 2376-2378(1990).

100 µl of suspensions in an aqueous 0.5 Tween 80 solution containing incarbillateine (Sample No. 9) so obtained, prepared to correspond to doses of 1, 2, 5 or 10 mg/kg of Sample No. 9, respectively, were intraperitoneally administered to mice and subjected to the formalin test in the same manner as in Example 1. Results (n=10, average ± standard deviation) are shown in the following Table 2 and Fig. 2. On the other hand, the same volume of an aqueous 0.5% Tween 80 solution was intraperiton eally administered to the control group.

**Table 2**

| Dose(mg/kg) | First phase of 0∼10 min. (sec.) | Second phase of 10∼30 min. (sec.) |
|---|---|---|
| Control | 131.46±7.84 | 164.50±19.68 |
| 1 | 108.21±5.53 * | 125.68±18.12 |
| 2 | 105.33±4.80 ** | 145.81±15.37 |
| 5 | 99.90±5.97 ** | 68.43±19.44 *** |
| 10 | 71.21±7.16 *** | 6.11±1.68 *** |

| | | |
|---|---|---|
| * P<0.05 | | |
| ** P<0.01 | | |
| *** P<0.001 | | |

### MEASUREMENT OF MOTOR ACTIVITY(QUANTITY OF VOLUNTARY MOTION)

An apparent analgetic and anti-inflammatory action may occur due to an action to suppress a motor activity. Thus, the motor activity was measured in the following manner:
Mice were left free in an apparatus for measuring a motor activity (Supermex, made by Muromachi Kikai K.K., Japan) for 30 minutes before the start to test just to adapt to test circumstances, then administered with the test drugs. 10 minutes after the drug administration, motor activity of mice was measured by the apparatus continuously for 30 minutes with time segments each of 5 minutes.

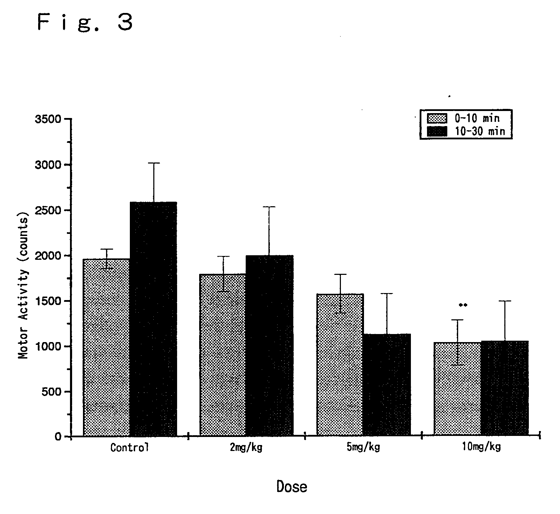

Test drugs (Sample No. 9) used were 100 µl of suspensions in an aqueous 0.5% Tween 80 solution containing Sample No. 9, prepared to correspond to doses of 2, 5 or 10 mg/kg of Sample No. 9, respectively, and intraperitoneally administered to mice. On the other hand, the same volume of an aqueous 0.5% Tween 80 solution was intraperitoneally administered to the control group. Results (n=10, average ± standard deviation) are shown in the following Table 3 and Fig. 3.

**Table 3**

| Dose(mg/kg) | First phase of 0∼10 min. (counts) | Second phase of 10∼30 min. (counts) |
|---|---|---|
| Control | 1964.40±106.47 | 2585.30±427.26 |
| 2 | 1792.20±194.83 | 1995.40±536.98 |
| 5 | 1568.40±215.64 | 1121.40±451.38 |
| 10 | 1027.30±247.09 ** | 1041.50±443.44 |

| | | |
|---|---|---|
| ** P<0.01 | | |

## Claims

1. An analgetic agent, which comprises an organic solvent or water extract of Incarvillea sinensis Lam. as an effective component.

2. An analgetic agent according to Claim 1, wherein the organic solvent extract is a methanol extract.

3. An analgetic agent according to Claim 1, wherein the organic solvent extract is a concentrate from eluate fractions obtained by subjecting a methanol extract to column chromatography using polystyrene-based resin, followed by elution of aqueous methanol solutions at various methanol concentrations.

4. An analgetic agent according to Claim 3, wherein the concentrate is from a 60% methanol eluate fraction.

5. An analgetic agent, which comprises incarvillateine or other monoterpene alkaloid extracted from Incarvillea sinensis Lam. as an effective component.

6. An analgetic agent according to Claim 5, wherein the other monoterpene alkaloid extracted from Incarvillea sinensis Lam. is incarvilline, oxyincarvilline, incarvine A or its N-oxide, incarvine B, incarvine C, incarvine D, incarvillateine N-oxide or 3''-methoxyincarvillateine.

7. An analgetic agent, which comprises an organic extract of Incarvillea sinensis Lam. containing incarvillateine or other monoterpene alkaloid extracted from Incarvillea sinensis Lam. as an effective component.

8. An analgetic agent according to Claim 7, wherein the other monoterpene alkaloid extracted from Incarvillea sinensis Lam. is incarvilline, oxyincarvilline, incarvine A or its N-oxide, incarvine B, incarvipe C, incarvine D, incarvillateine N-oxide or 3''-methoxyincarvillateine.
